Europäisches Patentamt

European Patent Office

Office européen des brevets

(19)

(11) Publication number: **0 184 629 B1**

# EUROPEAN PATENT SPECIFICATION

(12)

(45) Date of publication of patent specification: **18.12.91**

(51) Int. Cl.⁵: **A61L 25/00, A61F 5/44,**
//A61L29/00

(21) Application number: **85112730.8**

(22) Date of filing: **08.10.85**

(54) **Urine drainage bag outlet with barrier against microbial infection.**

(30) Priority: **12.12.84 US 680922**

(43) Date of publication of application:
**18.06.86 Bulletin 86/25**

(45) Publication of the grant of the patent:
**18.12.91 Bulletin 91/51**

(84) Designated Contracting States:
**DE FR IT SE**

(56) References cited:
FR-A- 2 546 056
US-A- 4 232 677
US-A- 4 372 313
US-A- 4 381 380

(73) Proprietor: **C.R. BARD, INC.**
**731 Central Avenue**
**Murray Hill New Jersey 07974(US)**

(72) Inventor: **Lee, Clarence C.**
**2 Cambridge Way**
**Princeton Jct. N.J. 08850-1111(US)**

(74) Representative: **Speidel, Eberhardt**
**Postfach 1320 Waldpromenade 26**
**W-8035 Gauting(DE)**

## Description

## BACKGROUND OF THE INVENTION

This invention relates to a urinary drainage system for a urinage drainage bag according to the preamble of claim 1.

## BRIEF DESCRIPTION OF THE PRIOR ART

Urine drainage bags are routinely used by post-operative patients as well as those with urological disorders. In practice, the patient is catherized and the catheter then connected to the drainage bag through a length of plastic tubing. The bag is normally supported below the level of the patient and the urine drains by gravity from the patient through the catheter, the tubing and then into the bag via a drip chamber. The bag may be emptied from time to time by means of the outlet tube which is normally closed to prevent leakage. The tube may discharge the contents of the bag into any convenient receptacle and then the outlet tube is clamped and the bag reused for the same patient.

The catheterized urinary track is one of the most common sites of hospital-acquired infection and in fact accounts for almost thirty percent of such infections. A significant improvement in the prevention of catheter associated infection has been by use of what is known as closed sterile drainage system. Despite these advances, still over twenty percent of patients with indwelling catheters continue to acquire urinary infections. Urine collection bags must be emptied at frequent intervals usually at least once every shift and the removal of bacterially contaminated urine can lead to the spread of urine infection. In order to minimize cross-contamination, the collected urine must be maintained in sterile condition during the collection period, even when the urine has a high bacterial count when it enters the drainage bag.

Despite the use of the most careful aseptic techniques almost fifty percent of catheterized patients develop an infection when the catheter is in place for twenty-four hours, and approximately ninety-eight percent or even more develop an infection after four days of use of such catheters. This is of course quite harmful to the patients and sujects them to the risk of cystitis and life threatening septicemia. The above-noted infections occur due to may circumstances. These include prolonged use of indwelling Foley-type catheters which are often accompanied by absence of sterile insertion and maintenance techniques, having the catheter connected to clean but not sterilized drainage collection containers, and others. The presence of urinary pathogens in the container which multiply and enter the urinary track through the ascending catheter which is a major pathway of infection is quite important. Various attempts have been made to reduce the migration of bacteria through the closed system including the bag, the drip chamber and the tubing connected to the catheter.

US-A-3 332 442 employs a connector between a catheter and a urine drainage bag for preventing movement of the bacteria from the bag to the patient. US-A-4 236 517, 4 193 403 and 4 241 733 show a dispensing device which releases paraformaldehyde to control the multiplication of pathogenes and prevent migration in catheters. US-A-4 233 263 teaches adding of hydrogen peroxide solution periodically to a urine bag for prevention of bacterial growth. Attempts have been made to provide a one-way inlet valve into the urine bag to prevent up migration, see US-A-3 312 22 and 4 323 677.

Other attempts have been made to treat the catheter itself with a microbicidal substance, see US-A-3 598 127, 3 566 874 and 3 695 921 which relate to an antibiotic material in a hydrophilic catheter coating.

US-A-4 417 892 describes a method for releasing a microbicidal substance by means of a frangible capsula which is inserted into the outlet drainage tube. The capsule must be broken in order to release the active agent.

US-A-4 372 313 describes a urinary receptacle whereby a ring of porous material such as foam that has been treated with antimicrobial agent is positioned in a hood in a tubular section which is located past the collecting receptacle. The ring of open-cell foam is soaked with an antiseptic solution and permitted to dry out. Urine which contacts the ring activates the antimicrobial agent in the ring in order to kill bacteria in the droplets of urine. However, the antimicrobial agent is not released in timed sequence for an extended period of time so that this device is effective only once in practive.

The same is true for the microbe-barrier drainage device of the afore-mentioned US-A-4 232 677 which provides a sponge impregnated with antiseptic substance.

US-A-4 381 380 discloses a thermoplastic urethane article treated with iodine for antibacterial use whereby the polyurethane polymers can range from a very weak film to an essentially rigid structure.

FR-A-2 546 056 focusses on a solid antiseptic agent in various physical shapes and adapters which can physically confine the agent. The adapters are arranged at both the upstream and downstream sides of the drainage bag. The antiseptic agent is in solid form and is dissolved by urine at its surface. As it dissolves, it shrinks or breaks apart and then the particles or fragments are flushed out the adapter by the urine. Therefore there is no controlled relase of the agent over a prolonged period of time.

It is the object of the invention to provide a urinary drainage system acoording to the preamble of claim 1 whereby the microbicidal substances are released in a timed sequence for an extended period of time, thus creating an effective barrier against migration of infectious organisms into the catheter.

According to the invention the porous member comprises an indenpendently formed microbicidal tube of a porous polymeric material housed in and connected to the interior of the outlet tube and containing a hydrophilic polymer which in response to contact with urine swells causing leaching out of the microbicidal substance.

This microbicidal tube is capable of providing antimicrobial activity for at least two weeks and as long as twenty days in actual practice. When the bag is drained the active ingredient is wetted by the urine and slowly dissolves and diffuses through the tubing material and is released uniformly at the surface of the tube thereby providing the desired barrier.

Preferably the tube consists of polypropylene and the hydrophilic polymer is D-3 polyethylene glycol polyurethane.

The microbicidal substance are preferably selected from the groups listed in claim 4.

The invention also comprises a microbicidal tube as claimed in in any of claims 1 to 4 for use in the outlet tube of a urinary drainage bag, the controllable sustained time release mechanism being activated upon contact with droplets of urine.

The microbicidal tube is easy to prepare using readily available materials and microbicidal substances. The prolonged effectiveness of the proposed tube obviates the need for frequent draining of bags, thus saving on nurse's time. The passsive, self-actuating release likewise is a labor-saving aspect. Since the need for human handling is substantially reduced, there is less of a chance for infection due to such contact.

BRIEF DESCRIPTION OF THE DRAWINGS

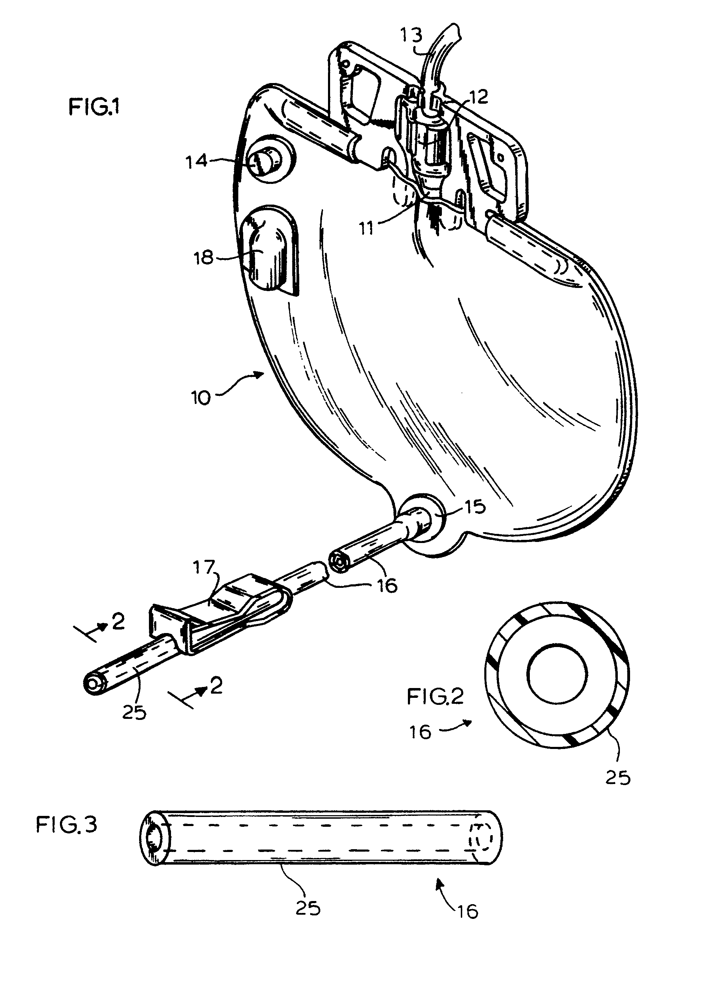

Fig. 1 is a perspective view of a conventional urine drainage bag and showing the outlet tube in broken apart fashion.

Fig. 2 is an enlarged cross section of the outlet tube taken along the lines 2-2 of Fig. 1.

Fig. 3 is a perspective view of a typical microbicidal tube used in the outlet tube.

DETAILED DESCRIPTION OF THE INVENTION

Referring now to the drawings, a conventional closed system urine drainage bag is shown generally at 10 and is formed by peripherally heat sealing or otherwise securing a pair of flat vinyl or PVC sheets. The bag is provided with an inlet 11 adjacent the top thereof for reception of a conventional drip chamber 12 and its associated tubing 13 which connects to a catheter which in turn is inserted in the urethral canal of the patient. An air vent and bacterial filter 14 is conventionally provided on one face of the bag.

The bag also includes a drain 15 terminating in an outlet tube or conduit 16 which may be formed of latex or any other suitable material, and which may be clamped off when not in use in a well known manner by means of the spring pinch clamp or valve 17 which is received about the outlet tube. The free end of the outlet is received in a protective housing 18 heat sealed to one face of the urine drainage bag.

Part of the outlet tube 16 houses a microbicidal tube 25. The microbicidal tube is typically 3,75 cm in length, 5.5mm in internal diameter (I.D.), 8.5mm in outer diameter (O.D.) and is 70% void. It is hollow inside in order to permit unimpeded urine flow. Obviously, the geometry and dimension of the microbicidal tube may be varied over wide limits yet shall function as a microbial barrier while permitting urine flow.

In general, the microbicidal tube is prepared by impregnating porous polymeric material with at least one microbicidal agent. Various kinds of polymeric materials can be used, but they must be crystalline and have a high melting point which will allow them to withstand exposure to body fluid temperatures without softening. Usually the tube is made by one of three methods. 1) A porous material, such as polypropylene is impregnated with at least one microbicidal agent. It is then coated with a hydrophilic polymer which in response to contact with urine swells, causing the leaching out of the microbicidal substance. 2) A porous material, such as high density polyethylene is impregnated with a hydrophilic polymer and at least one microbicidal agent. 3) The microbicidal tube is made by compounding and co-extruding a polymer, such as silicone, with at least one microbicidal agent, and then coated with a hydrophilic polymer.

The solution of the additives and the polymeric material is allowed to react for a suitable length of time in the presence of solvent or solvents. The usual solvents in the preparation of the microbicidal tube are ethanol and dimethyl sulfoxide. At the end of the reaction time, the microbicidal tube is dried by conventional methods and sterilized with ethylene oxide (ETO).

The specific antimicrobial substance to be used is left to the choice of the manufacturer, however such substance must readily be compounded into polymers or adhere to the porous polymeric material which in turn will absorb the substance. The biocidal additives can be selected from a very large group of commercially available antibiotics, drugs, antiseptics. Some examples of the common active agents that can be incorporated into the microbicidal tube are: penicillin, tetracycline, triclosan, nalidixic acid, sulfamylon, amphotericin B, norfloxacin, haloprogin, gentamicin, chlorhexidine, clotrimazole, tolnaftate, polymixin, parachlorometaxylenol, pyrithione, hexachlorophene, nitrofurazone, nitrofurantoin, chlorixin. Microbicidal agents may be incorporated either singly or in various combinations.

The microbicidal tube may be inserted inside the outlet tube during normal manufacturing conditions and there will be no loss of the biocidal activity since the substance does not become released until it comes in contact with urine. The microbicidal tube is effective for at least two weeks. During this time, it continues to release in a timed sequence, the microbicidal agents, thus creating an effective barrier against upward movement and multiplication of organisms and the subsequent infection of the urinary tract.

The amount of drug released will depend on a number of factors, such as for example, the specific biocidal agent used, the length of time it is desired to release the drug, the dosage that is to be administered in a specific time, etc. The dosage can be controlled by varying the concentrations (or amounts) of the drug(s) and hydrophilic polymer and physical parameters such as pore size and shape of the support polymer used.

There are a number of important advantages that the microbicidal tube offers over the apparatus and methods of prior art.

Thus, the prolonged effectiveness of the microbicidal tube (two weeks at least) saves on nurses' time, for the bag need not be drained as often as it has to be, using prior art apparatus. The passive nature of the sustained drug release, activated upon contact with urine, likewise saves on the nurse's time, for there is no necessity for human participation. Moreover, this is likewise a more reliable and certain method of controlling and preventing infections than methods requiring periodic handling of apparatus. Such periodic manipulation is frequently delayed or entirely disregarded. Additionally, the less human handling that is involved, the less is there a chance for contamination from the outside, hence the decrease in an opportunity for an infection.

The following examples describe the manner and process of making and using the invention and represent the best mode contemplated by the inventor.

Preparation of Microbicidal Tube

Example 1

Batchwise compound 85% polypropylene, 10% hexachlorophene, 4% gentamicin A HCl and 1% clotrimazole at 196°C and, then, extrude in into a tube. A 2.5cm segment of the tube is dipped into a solution of 96% ethanol and 4% D-3 polyethylene glycol polyurethane (D-3) for 5 seconds, air-dried at room temperature (RT) for 10 minutes and oven dried at 85°C for 10 minutes.

Example 2

Batchwise compound 88% polypropylene, 6% triclosan, 4% chloroxine, and 2% tolnaftate at 198°C and then extrude it into a tube (5.5mm ID/8.5mm OD). A 3.75cm segment of the tube is dipped into a solution containing 96.5% ethanol and 3.5% D-3 for 5 seconds, air-dried at RT for 10 minutes and oven-dried at 52°C for 30 minutes.

Example 3

Batchwise compound 91% polythylene, 6% nalidixic acid and 3% parachlorometaxylenol at 150°C to a homogenous dispersion and extrude it into a tube (6.5mm ID/8.5mm OD). A 2.5cm segment of the tube is dipped in a solution containing 97% ethanol and 3% D-3 for 5 seconds, air-dried at RT for 10 minutes and oven-dried at 78°C for 10 minutes.

Example 4

Batchwise compound 89.8% polyethylene, 8% zinc pyrithione and 2.2% tetracycline HCl at 152°C to a homogenous dispersion and extrude it into a tube (6.5mm ID/8.5mm OD). A 3.75cm segment of it is dipped in a solution of 95% ethanol and 5% D-3 for 5 seconds, air-dried at RT for 10 minutes and oven-dried at 65°C for 10 minutes.

Example 5

A segment of microporous polysulfone tube is immersed in a solution of 88.8% ethanol, 7% gentamicin A HCl, 4% D-3 polyethylene glycol polyurethane and 0.2% zinc pyrithione for 10 minutes. It is then air-dried at room temperature (RT) for 10 minutes and oven-dried at 70°C for 15 minutes. The concentration of gentamicin A HCl is below its saturation point and could be raised if wanted. Zinc pyrithione, an antifugal agent, is approximately at its optimum concentration. It could be replaced by the more soluble sodium pyrithione.

Example 6

A segment of microporous polysufone tube is immersed in a solution of 79.5% ethanol, 11% tetracycline HCl, 5.3% polymixin B HCl, 3.5% D-3 polyethylene glycol polyurethane and 0.2% zinc pyrithione for 10 minutes. It is air-dried at RT for 10 minutes and oven-dried at 68°C for 20 minutes. Both tetracycline and polymixin are below their saturation points.

Example 7

A segment of microporous high density polyethylene tube (HDPE; pore size: 50 microns) is immersed in a solution of 50% anhydrous acetone, 40% anhydrous ethanol, 5% gentamicin HCl, and 5% parachlorometaxylenol for 5 minutes. It is air-dried at RT for 15 minutes, dipped in 96.5% ethanol, 3.5% D-3 polyethylene glycol polyurethane for 5 seconds, air-dired at RT for 10 minutes and oven-dried at 70°C for 15 minutes. Concentrations of D-3, gentamicin and parachlorometaxylenol could be increased if needed.

Example 8

A segment of microporous HDPE is immersed in a solution of 73% ethanol, 10% tetracycline HCl, 10% parachlorometaxylenol, 3.8% D-3, 2% dimethyl sufoxide (DMSO), 0.6% clotrimazole and 0.6% tolnaftate for 5 minutes. It is then air-dried at RT for 10 minutes, oven-dried at 55°C for 20 minutes, and air-dried at RT for 18 hours.

Example 9

A segment of HDPE tube is immersed in a solution of 41.4% DMSO, 44.6% acetone, 10% triclosan and 4.3% chloroxine for 10 minutes. It is air-dried at RT for 15 minutes, dipped into 96.5% ethanol and 3.5% D-3 for 5 seconds, air-dried at RT for 10 minutes and oven-dried at 55°C for 30 minutes. Concentrations of triclosan and chloroxine could be raised substantially if needed.

Example 10

A segment of microporous polypropylene tube (5.5mm ID/8.5mm OD; 70% void) is immersed in a solution of 83.5% anhydrous acetone, 8% chlorhexidine acetate, 5.5% triclosan and 3% Hypol® 3000 polyurethane for 5 minutes. It is air-dried at RT for 5 minutes, oven-dried at 52°C for 10 minutes and air-dried at RT for 18 hours. Concentrations of chlorhexidine and triclosan can be increased if needed.

Example 11

A segment of microporous polypropylene tube is immersed in a solution containing 50% ethanol, 33% chloroform, 11% hexachlorophene, 3.8% D-3, 1% nalidixic acid, 0.6% clotrimazole and 0.6% tolnaftate for 10 minutes. It is air-dried at RT for 10 minutes and oven-dried at 72°C for 25 minutes. Concentrations of clotrimazole and tolnaftate are substantially below their saturation points in the solvent system.

Example 12

A segment of microporous polypropylene tube is immersed in a solution of 46.2% ethanol, 22% DMSO, 11% parachlorometaxylenol, 11% dichlorhexidine diacetate, 4.8% water, 4.0% D-3 and 1.0% nitrofurazone for 10 minutes. It is air-dried at RT for 10 minutes, oven-dried at 68°C for 10 minutes and air-dried at RT for 18 hours. The concentration of parachlorometaxylenol could be increased if needed.

Example 13

A segment of microporous polypropylene tube is immersed in a solution containing 84% ethanol, 7.5% hexachlorophene, 5% D-3, 2.5% DMSO, 0.7% $H_2O$, 0.25% clotrimazole and 0.05% aminacrine for 10 minutes. It is air-dried at RT for 10 minutes, oven-dried at 78°C for 20 minutes and air-dried at RT for 18 hours.

Example 14

A segment of microporous polypropylene tube is immersed in a solution of 50.5% ethanol, 31% acetone, 10% hexachlorophene, 4.3% D-3, 2.5% DMSO, 1% clotrimazole an d0.5% $H_2O$ for 10 minutes. It is air-dried at RT for 10 minutes, oven-dried at 78°C for 20 minues, and air-dried at RT for 18 hours.

Example 15

A segment of microporous polypropylene tube is immersed in a solution containing 44.1% ethanol, 31% DMSO, 11% triclosan, 9% hexachlorophene, 3.4% D-3, 1.0% clotrimazole, and, 0.5% nitrofurazone for 10 minutes. It is air-dried at RT for 15 minutes, oven-dried at 52°C for 30 minutes and air-dried at RT for 18 hours. Concentrations of triclosan, hexachlorophene and clotrimazole could be increased if needed.

The foregoing preparations of microbicidal tubes were effective in maintaining sterility for 17 to 21 days upon exposure to urine.

## Claims

1. A urinary drainage system for a urinage drainage bag (10), comprising an outlet tube (16) secured to the bottom part of said drainage bag and comprising a porous member for storage and sustained time release of at least one microbicidal substance, characterized in that said member comprises an independently formed microbicidal tube (25) of a porous polymeric material housed in and connected to the interior of said outlet tube (16) and containing a hydrophilic polymer which in response to contact with urine swells causing the leaching out of the microbicidal substance.

2. The urinary drainage system according to claim 1 characterized in that said tube (25) consists of polypropylene.

3. The urinary drainage system according to claim 1 or 2, characterized in that the hydrophilic polymer is D-3 polyethylene glycol polyurethane.

4. The urinary drainage system according to any of claims 1 to 3, characterized in that said microbicidal substances are selected from any of the following groups:
   a) hexachlorophene, gentamycin and clotrimazole,
   b) triclosan, chloroxine and tolnaftate,
   c) nalidixic acid and parachlorometaxylenol,
   d) zinc pyrithione and tetracycline,
   e) gentamycin and zinc pyrithione,
   f) tetracycline polymixin and zinc pyrithione,
   g) gentamycin and zinc pyrithione,
   h) tetracycline, parachlorometaxylenol, clotrimazole and tolnaftate,
   i) triclosan and chloroxine,
   j) chlorhexidine and triclosan,
   k) hexachlorophene, nalidixic acid, clotrimazole and tolnaftate,
   l) parachlorometaxylenol, chlorhexidine and nitrofurazone,
   m) hexachlorophene, clotrimazole and aminacrine,
   n) hexachlorophene and clotrimazole,
   o) triclosan, hexachlorophene, clotrimazole and nitrofurazone.

5. The microbicidal tube according to any of claims 1 to 4 for use in the outlet tube (16) of a urine drainage bag (10), the controllable sustained time release mechanism being activated upon contact with droplets of urine.

## Revendications

1. Système de drainage urinaire pour un sac (10) de drainage urinaire, comportant un tuyeau d'écoulement (16) fixé à la partie inférieure dudit sac de drainage et un élément poreux pour le stockage et le débit continu de quelque temps au moins d'une substance microbicide, caractérisé en ce que ledit élément comprend un tuyeau microbicide (25) d'une forme indépendante en matériel poreux polymérique logé dans et raccordé à l'intérieur dudit tuyeau d'écoulement (16) et contentant un polymère hydrophile qui, sous l'effet du contacte avec l'urine, se gonfle et permet à la substance microbicide de s'écouler.

2. Système de drainage urinaire suivant la revendication 1; caractérisé en ce que le tuyeau (25) est en polypropylène.

3. Système de drainage urinaire suivant l'une des revendications 1 ou 2, caractérisé en ce que le polymère hydrophile est D-3 polythène glycol polyurethane

4. Système de drainage urinaire suivant l'une quelconque des revendications 1 à 3, caractérisé en ce que les substances microbicides sont choisies dans une quelconque des groupes suivantes:
   a) hexachlorophène, gentamycine et clotrimazole,
   b) triclosane, chloroxine et tolnaftate,
   c) acide nalidixique et parachlorometaxylenol,
   d) pyrithione de zinc et tetracycline
   e) gentamycine et pyrithione de zinc
   f) tetracycline et pyrithione de zinc,
   g) gentamycin et pyrithione de zinc,
   h) tetracycline, parachlorometaxylenol, clotrimazole et tolaftate
   i) triclosane et chloroxine,
   j) chlorhexidine et triclosane,
   k) hexachlorophène, acide nalidixique, clotrimazole et tolnaftate
   l) parachlormetaxylenol, chlorhexidine et nitrofurazone,
   m) hexachlorophène, clotrimazole et aminacrine,
   n) hexachlorophène et clotrimazole,
   o) triclosane, hexachlorophène, clotrimazole et nitrofurazone.

5. Tuyeau microbicide suivant l'une quelconque des revendications 1 à 4, destiné à utilisation dans le tuyeau d'écoulement (16) d'un sac de drainage urinaire (10), le mécanisme de débit continu de quelque temps contrôlable étant actionné au moment du contact avec des gouttes d'urine.

**Patentansprüche**

1. Urin-Drainagesystem für einen Urin-Drainagebeutel (10) mit einem Auslaßrohr (16), das am Bodenteil des Beutels angebracht ist und ein poröses Teil zum Einlagern mindestens einer antibiotischen Substanz und zur kontinuierlicher Abgabe derselben über einen längeren Zeitraum aufweist, dadurch gekennzeichnet, daß das Teil ein unabhängig gebildetes antibiotisches Rohr (25) aus einem porösen Polymer-Material aufweist, das im Inneren des Auslaßrohres (16) angeordnet und mit diesem verbunden ist und ein hydrophiles Polymer enthält, das bei Kontakt mit Urin anschwillt und das Ausschwemmen der antibiotischen Substanz bewirkt.

2. Urin-Drainagesystem nach Anspruch 1; dadurch gekennzeichnet, daß das Rohr (25) aus Polypropylen besteht.

3. Urin-Drainagesystem nach Anspruch 1 oder 2, dadurch gekennzeichnet, daß das hydrophile Polymer D-3 Polyethylenglykolpolyurethan ist.

4. Urin-Drainagesystem nach einem der Ansprüche 1 bis 3, dadurch gekennzeichnet, daß die antibiotische Substanzen aus einer der folgenden Gruppen ausgewählt sind:
   a) Hexachlorophen, Gentamycin und Clotrimazol
   b) Triclosan, Chloroxin (5,7 Dichlor-8-hydroxyquinolin) und Tolnaftat
   c) Nalidixinsäure und p-Chlor-m-xylenol
   d) Pyrithion-Zink und Tetracyclin
   e) Gentamycin und Pyrithion-Zink
   f) Tetracyclinpolymyxin und Pyrithion-Zink
   g) Gentamycin und Pyrithion-Zink
   h) Tetracyclin, p-Chlor-m-xylenol, Clotrimazol und Tolnaftat
   i) Triclosan und Chloroxin
   j) Chlorhexidin und Triclosan
   k) Hexachlorophen, Nalidixinsäure, Clotrimazol und Tolnaftat
   l) p-Chlor-m-xylenol, Chlorhexidin und Nitrofural
   m) Hexachlorophen, Clotrimazol und Aminoacridin
   n) Hexachlorophen und Clotrimazol
   o) Triclosan, Hexachlorophen, Clotrimazol und Nitrofural.

5. Antibiotisches Rohr nach einem der Ansprüche 1 bis 4 zur Verwendung im Auslaßrohr (16) eines Urin-Drainagebeutels (10), wobei der kontrollierbare Mechanismus zur kontinuierlichen Abgabe über einen längeren Zeitraum bei Kontakt mit Urin-Tröpfchen aktiviert wird.

FIG.1

14

18

10

13

12

11

15

17

16

2

2

25

FIG.2

16

25

FIG.3

25

16